(19) Europäisches Patentamt
European Patent Office
Office européen des brevets

(11) Veröffentlichungsnummer: **0 031 456**
**B1**

# (12) EUROPÄISCHE PATENTSCHRIFT

(45) Veröffentlichungstag der Patentschrift: **18.04.84**

(51) Int. Cl.[3]: **C 07 D 335/02**, C 07 D 409/04, C 07 D 409/14, A 61 K 31/38, A 61 K 31/445, A 61 K 31/495

(21) Anmeldenummer: **80107291.9**

(22) Anmeldetag: **22.11.80**

(54) **Neue substituierte 4-Amino-2,6-diaryl-tetrahydrothiopyrane, deren Säureadditionssalze, Verfahren zu ihrer Herstellung und pharmazeutische Zusammensetzungen.**

(30) Priorität: **21.12.79 DE 2951634**

(43) Veröffentlichungstag der Anmeldung: **08.07.81 Patentblatt 81/27**

(45) Bekanntmachung des Hinweises auf die Patenterteilung: **18.04.84 Patentblatt 84/16**

(84) Benannte Vertragsstaaten: **AT BE CH DE FR GB IT LI LU NL SE**

(56) Entgegenhaltungen:
**DE - A - 1 543 840**
**US - A - 3 787 442**

**Die Akte enthält technische Angaben, die nach dem Eingang der Anmeldung eingereicht wurden und die nicht in dieser Patentschrift enthalten sind.**

(73) Patentinhaber: **C.H. BOEHRINGER SOHN, Postfach 200, D-6507 Ingelheim am Rhein (DE)**

(72) Erfinder: **Weber, Karl-Heinz, Dr., Kaiser-Karl-Strasse 11, D-6535 Gau-Algesheim (DE)**
Erfinder: **Schneider, Claus, Dr., Tannenweg 1, D-6507 Ingelheim/Rhein (DE)**
Erfinder: **Walter, Gerhard, Dr., Pfarrer-Heberer-Strasse 37, D-6530 Bingen/Rhein (DE)**
Erfinder: **Pook, Karl-Heinz, Dr., Tannenweg 22, D-6507 Ingelheim/Rhein (DE)**
Erfinder: **Böke, Karin, Dr., Selzstalstrasse 99, D-6507 Ingelheim/Rhein (DE)**
Erfinder: **Bechtel, Wolf Dietrich, Dr., Mühlstrasse 3, D-6531 Appenheim (DE)**

**EP 0 031 456 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Neue substituierte 4-Amino-2,6-diaryl-tetrahydrothiopyrane, deren Säureadditionssalze, Verfahren zu ihrer Herstellung und pharmazeutische Zusammensetzungen

Die vorliegende Erfindung betrifft substituierte 4-Amino-2,6-diaryl-tetrahydrothiopyrane, deren Säureadditionssalze, Verfahren zu ihrer Herstellung und pharmazeutische Zusammensetzungen.

Die neuen Verbindungen besitzen die allgemeinen Formeln

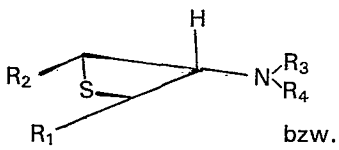

(I)

bzw.

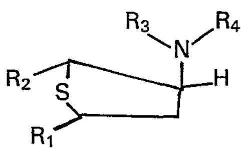

(Ia)

worin die Reste

$R_1$ und $R_2$, die gleich oder verschieden sein können, Phenyl, ein-, zwei- oder dreifach durch ein Halogenatom, eine Methyl- oder Methoxygruppe substituiertes Phenyl, Thienyl oder Furyl bedeuten,

$R_3$ für Wasserstoff oder eine geradkettige oder verzweigte Alkylgruppe mit 1-3 Kohlenstoffatomen und

$R_4$ für eine geradkettige oder verzweigte Alkylgruppe mit 1-3 Kohlenstoffatomen steht oder beide Reste $R_3$ und $R_4$ zusammen mit dem Stickstoffatom einen Piperidin-, Piperazin- oder Morpholinoring bilden, wobei der Piperidinring gegebenenfalls in 4-Stellung durch eine Amino- oder Dimethyl-Aminogruppe weiter substituiert ist oder der Piperazinring in 4-Stellung eine Alkylgruppe mit 1-2 Kohlenstoffatomen aufweisen kann.

Die neuen Verbindungen können erhalten werden durch Reduktion der entsprechenden Tetrahydrothiopyranone der allgemeinen Formel

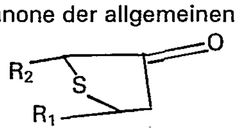

(II)

worin $R_1$ und $R_2$ die oben angeführte Bedeutung besitzen, mittels geeigneter Reduktionsmittel, wie Lithiumaluminiumhydrid, Natriumborhydrid oder Natriumcyanoborhydrid.

Man erhält die beiden entsprechenden stereoisomeren Carbinole der allgemeinen Formeln

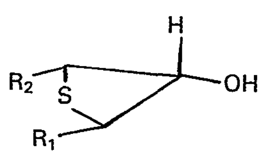

bzw.

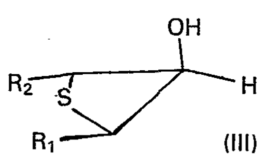

(III) (IIIa)

worin die Reste $R_1$ und $R_2$ die oben genannte Bedeutung besitzen. Diese Carbinole werden in üblicher Weise verestert, vorzugsweise mit einem p-Toluolsulfonylhalogenid oder einem Methansulfonsäurehalogenid; der auf diese Weise erhaltene Ester wird anschliessend mit einem primären oder sekundären Amin umgesetzt.

Zur Herstellung solcher Endprodukte, die eine Mono- oder Dialkylaminogruppe in 4-Stellung des Moleküls tragen, kann man ein Tetrahydropyranon der Formel II bei Temperaturen von 0 bis 10 °C mit einer Mischung aus Ameisensäure und einem entsprechenden Amin der allgemeinen Formel

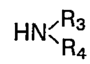

(IV)

worin

$R_3$ Wasserstoff oder eine geradkettige oder verzweigte Alkylgruppe mit 1-3 Kohlenstoffatomen und

$R_4$ eine geradkettige oder verzweigte Alkylgruppe mit 1-3 Kohlenstoffatomen bedeutet, umsetzen.

Die gleichen Endprodukte lassen sich aus den Tetrahydropyranonen der Formel II auch durch Umsetzung der entsprechenden Amine der Formel IV in Gegenwart von Wasserstoff und einem Katalysator wie Palladium oder Raney-Nickel oder in Gegenwart eines komplexen Hydrides, z.B. Natriumborhydrid, Lithiumaluminiumhydrid oder Natriumcyanoborhydrid, erhalten.

Die Herstellung der Endprodukte gelingt schliesslich auch durch übliche Alkylierung von Tetrahydrothiopyranen mit freier Aminogruppe der allgemeinen Formel

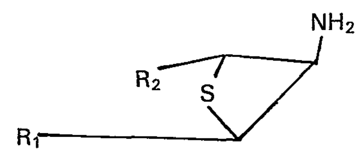

(V)

worin die Reste $R_1$ und $R_2$ die oben angeführte Bedeutung besitzen. Diese Verbindungen, die zum Teil aus der Arbeit von V. Baliah et al, Ind. J. Chem. Sec.B *16*, 1978, *9*, 776 bereits bekannt sind, sind durch Reduktion der entsprechenden Tetrahydrothiopyranon-oxime leicht zugänglich.

oxime leicht zugänglich.

Wie bereits aus den Formeln I und Ia hervorgeht, treten die Endprodukte in zwei stereoisomeren Formen auf;

die Aminogruppe in 4-Stellung des Moleküls kann äquatorial (e) oder axial (a) angeordnet sein.

Die räumlich anspruchsvollen Reste in 2- und 6-Stellung des Tetrahydrothiopyranringes sind, wie die Protonenspektren zeigen, äquitorial angeordnet.

Die Trennung der beiden Stereoisomeren erfolgt bevorzugt auf der Stufe des Endproduktes und wird auf bekannte Weise, entweder chromatographisch oder durch fraktionierte Kristallisation, durchgeführt.

Die Endprodukte der allgemeinen Formeln I bzw. Ia können gewünschtenfalls nach üblichen Methoden

in ihre physiologisch unbedenklichen Säureadditionssalze überführt werden.

Als Säuren eignen sich hierfür sowohl anorganische Säuren wie Halogenwasserstoffsäuren, Schwefelsäure, Phosphorsäure und Aminosulfonsäure als auch organische Säuren wie Ameisensäure, Essigsäure, Propionsäure, Milchsäure, Glykolsäure, Glukonsäure, Maleinsäure, Bernsteinsäure, Weinsäure, Benzoesäure, Salicylsäure, Zitronensäure, Ascorbinsäure, p-Toluolsulfonsäure oder Oxyäthansulfonsäure.

Die als Ausgangsstoffe eingesetzten Tetrahydrothiopyranone der allgemeinen Formel II können erhalten werden durch Umsetzung eines gegebenenfalls substituierten Benzalacetons (1) mit einem Aldehyd der Formel (2) und anschliessendem Ringschluss der so erhaltenen Verbindung (3) mit Schwefelwasserstoff in Alkohol/Natriumacetat nach folgendem Schema:

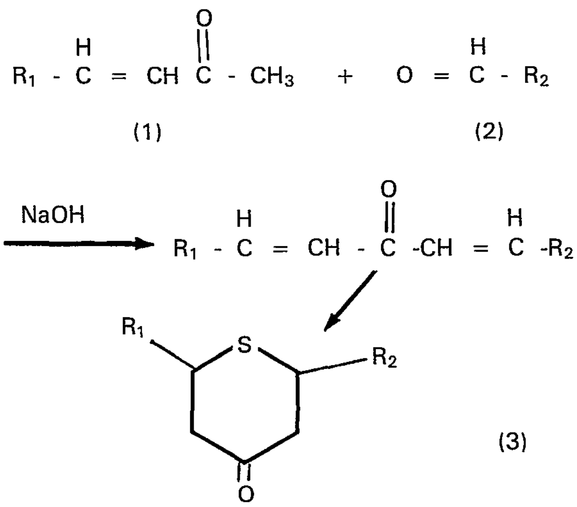

(1) (2)

(3)

Die neuen Stoffe der allgemeinen Formeln I und Ia stellen wertvolle Pharmazeutika dar. Aufgrund der bei der Messung des sogenannten «Re-Uptake» von Noradrenalin und Serotonin erhaltenen Werte kann erwartet werden, dass die neuen Verbindungen sehr gute antidepressive Eigenschaften besitzen.

Es ist bekannt, dass es bei verschiedenen Depressionsformen in bestimmten Gehirnarealen zu einer Verarmung an biogenen Aminen, vor allem an Noradrenalin und Serotonin, kommt; die biogenen Amine können dadurch vermehrt werden, dass der Re-Uptake in die Neuronen verhindert wird. Eine geeignete Versuchsanordnung zeigt nun, dass die axialen Isomeren eine grössere Hemmwirkung auf den Noradrenalin-Re-Uptake, die äquitorialen Isomeren auf den Serotonin-Re-Uptake ausüben.

Am wachen Ganztier haben sich die neuen Verbindungen ferner als gute Reserpinantagonisten erwiesen.

Als besonders wirksam seien die 4-Mono- bzw. Dialkylamino-Verbindungen hervorgehoben, bei denen festgestellt wurde, dass in der Reihe der 4a-Derivate die Toxizität in der Reihenfolge $R_1$, $R_2$ = Phenyl → substituiertes Phenyl → Heteroaryl abnimmt.

Unter diesen Verbindungen sind besonders diejenigen hervorzuheben, die in 2,6-Stellung einen substituierten Phenylring aufweisen, z.B. das 4a- und 4e-Dimethylamino-2,6-bis-(4-methoxyphenyl)-tetrahydrothiopyran, das 4a- und 4e-Methylamino-2,6-bis-(4-methoxyphenyl)-tetrahydrothiopyran und das 4a- und 4e-Dimethylamino-2-(4-chlorphenyl)-6-phenyl-tetrahydrothiopyran bzw. deren Säureadditionssalze.

Die Einzeldosis der erfindungsgemässen Substanzen liegt zwischen 0,2 und 50, vorzugsweise zwischen 0,5 und 25 mg (oral) und zwischen 5 und 150 mg als Tagesdosis.

Die erfindungsgemäss erhältlichen Verbindungen können allein oder in Kombination mit anderen erfindungsgemässen Wirkstoffen, gegebenenfalls auch in Kombination mit weiteren pharmakologisch aktiven Wirkstoffen wie Tranquilizern oder β-Rezeptorenblockern zur Anwendung gelangen. Geeignete Anwendungsformen sind beispielsweise Tabletten, Zäpfchen, Lösungen, Säfte, Emulsionen oder dispersible Pulver.

Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxypolymethylen, Carboymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemässen Wirkstoffe bzw. Wirkstoffkombinationen können zusätzlich noch ein Süssungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können ausserdem Suspendierhilfsstoffe oder Dickungsmittel, wie Natriumcarboxymethylcellulose, Netzmittel, beispielsweise Kondensationsprodukte von Fettalkoholen mit Äthylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Äthylendiamintetraessigsäure hergestellt und in Injektionsflaschen oder Ampullen abgefüllt.

Die eine oder mehrere Wirkstoffe bzw. Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol bzw. dessen Derivaten, herstellen.

Die folgenden Beispiele dienen zur näheren Erläuterung der Erfindung.

*Beispiel 1*

*4a- und 4e-Dimethylamino-2-(4-chlorphenyl)-6--phenyl-tetrahydrothiopyran*

0,036 Mol (11 g) 2-(4-Chlorphenyl)-6-phenyl-tetrahydrothiopyranon-(4) werden in 60 ml Dimethylformamid gelöst und bei 20°C eine Lösung von Dimethylaminoformiat (hergestellt aus 3,2 g Ameisensäure und 12,6 g Dimethylamin bei —10°C) zugetropft. Man kocht das Reaktionsgemisch unter Rühren 4 Stunden unter Rückfluss, kühlt die Lösung auf 10°C ab, versetzt mit 50 ml Äther und säuert vorsichtig mit 2n Salzsäure an (pH 2). Die wässrige Phase wird mit 50%iger Natronlauge neutralisiert und wiederholt mit Äther extrahiert. Nach dem Trocknen und Eindampfen der Ätherlösungen gibt man den Rückstand auf eine $SiO_2$-Säule und eluiert zunächst mit einer Mischung aus Methylenchlorid/Methanol 99:1. Dabei erhält man 3 g der 4a-Dimethylamino-Verbindung vom Fp. 115°C.

In dem Elutionsmittel wird jetzt der Anteil an Methanol auf 5% erhöht und man erhält schliesslich 1,2 g der 4e-Dimethylamino-Verbindung vom Fp. 69 bis 70°C. Die Ausbeute an 4a- und 4e-Dimethylaminoisomeren beträgt zusammen 35% d.Th.

Das als Ausgangsstoff eingesetzte Tetrahydrothiopyranon wird wie folgt hergestellt:

0,14 Mol (20 g) Benzalaceton und 0,14 Mol p-Chlorbenzaldehyd werden in 250 ml Äthanol gelöst und nach Zusatz von 20 ml 10%iger Natronlauge bei ca. 10°C 60 Minuten kräftig gerührt. Man saugt die ausgefallenen gelben Kristalle ab, wäscht mit wenig Wasser und kristallisiert aus Isopropanol um. Dabei erhält man 25,2 g (68,5% d.Th.) hellgelbe Kristalle vom Fp. 133°C. (Vgl. Verbindung 3 des Reaktionsschemas).

0,1 Mol (25 g) dieser Verbindung werden mit 0,34 Mol (27,8 g) Natriumacetat in 700 ml 90%igem Alkohol suspendiert. Unter Rühren und Rückflusskochen leitet man 4 Stunden einen kräftigen $H_2S$-Strom in die Lösung. Danach wird das Lösungsmittel abdestilliert, der Rückstand zwischen Wasser und Methylenchlorid verteilt, die Methylenchloridphase getrocknet und eingedampft. Den Rückstand gibt man auf eine $SiO_2$-Säule und eluiert mit Methylenchlorid. Die Eluate werden eingedampft und mit Isopropyläther zur Kristallisation gebracht. Man erhält 40% d.Th. (11,2 g) 2-(4-Chlorphenyl-6-phenyl-tetrahydrothiopyranon-(4) vom Fp. 124°C.

*Beispiel 2*

*4a- und 4e-(4-Methylpiperazino)-2,6-bis-(4-chlorphenyl)-tetrahydrothiopyran*

0,012 Mol (5 g) eines Gemisches von 4a- und 4e-Methansulfonyloxy-2,6-bis-(4-chlorphenyl)-tetrahydrothiopyran werden mit 10 ml 1-Methylpiperazin 12 Stunden unter Rückfluss gekocht. Man destilliert das überschüssige 1-Methylpiperazin ab, gibt den Rückstand auf eine $SiO_2$-Säule und eluiert mit Methylenchlorid/Methanol 99:1. Als erste Fraktion erhält man das 4a-Methylpiperazinylisomere als zähes Öl (1,4 g). Das hieraus mit alkoholischer Salzsäure hergestellte Hydrochlorid (1,3 g) schmilzt bei 286 bis 288°C. Durch Erhöhung des Methanolanteils auf 5% und weitere Elution erhält man die 4e-Titelverbindung vom Fp. 159°C als Base (1,1 g). Die Gesamtausbeute beträgt 40% d.Th.

Das Ausgangsmaterial wird wie folgt hergestellt:

In eine Lösung von 360 ml 10%ige wässrige Natronlauge und 280 ml Äthanoll gibt man bei Raumtemperatur zunächst die Hälfte eines Gemisches aus 50 g p-Chlorbenzaldehyd und 10,4 g Aceton (Molverhältnis 2:1) und rührt 15 Minuten. Dann wird die zweite Hälfte der Aldehyd-Ketonmischung zugefügt und weitergerührt. Nach 30 Minuten saugt man die gelben Kristalle ab, wäscht mit Wasser und kristallisiert aus wenig Isopropanol um. Die Ausbeute beträgt 50 g (91% d.Th.) vom Fp. 187-188°C. (Vgl. Verbindung 3 des Reaktionsschemas).

0,16 Mol (49 g) dieser Verbindung werden zusammen mit 43 g Natriumacetat in 1 l 10%igem Äthanol suspendiert und unter Rückflusskochen 4 Stunden $H_2S$ eingeleitet. Anschliessend wird eingeengt, mit Methylenchlorid ausgeschüttelt und die organische Phase getrocknet. Man dampft das Lösungsmittel ab und bringt den Rückstand durch Zugabe von Isopropyläther zur Kristallisation. Die Ausbeute beträgt 31 g (57% d.Th.) vom Fp. 141-143°C.

0,05 Mol (18 g) dieses Thiopyranons werden in 100 ml Methanol suspendiert. Unter Rühren gibt man bei 0°C 7,4 g (0,2 Mol) Natriumborhydrid portionsweise hinzu. Nach 2stündigem Rühren destilliert man das Lösungsmittel ab, verteilt den Rückstand zwischen Methylenchlorid und Wasser, trocknet die Methylenchloridphase und dampft ein. Zurück bleiben 13,1 g (72,4% d.Th.) 2,6-Bis-(4-chlorphenyl)-tetrahydrothiopyran-4a- und 4e-carbinol vom Fp. 194-195°C.

13 g (0,04 Mol) des Carbinolgemisches werden mit 6,9 g (0,06 Mol) Methansulfonsäurechlorid in 100 ml Methylenchlorid gelöst. Man fügt 4,8 g Pyridin hinzu und kocht 16 Stunden unter Rückfluss. Nach dem Abkühlen wird das überschüssige Methansulfonsäurechlorid mit Wasser und Ammoniak vorsichtig zersetzt. Die Methylenchloridphase wird abgetrennt, mit Wasser gewaschen und getrocknet. Nach dem Eindampfen gibt man den Rückstand auf eine $SiO_2$-Säule und eluiert mit Methylenchlorid. Aus dem Eluat werden schliesslich 14,1 g (88% d.Th.) des Esters vom Fp. 196-197°C erhalten.

Analog den Beispielen 1 und 2 wurden ferner die folgenden Verbindungen erhalten:

| Beispiel Nr. | $R_1$ | $R_2$ | $R_3$ | $R_4$ | Stellung d. Amino-gruppe | Fp. °C |
|---|---|---|---|---|---|---|
| 3 | 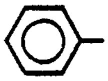 | 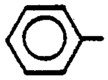 | $-CH_3$ | $-CH_3$ | a | Base 68-70 HCl-Salz 204-205 |
| 4 | 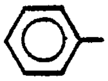 | 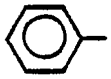 | $-CH_3$ | $-CH_3$ | e | 96-104 |
| 5 | 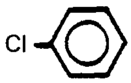 | 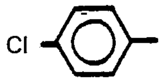 | $-CH_3$ | $-CH_3$ | a | 158-159 |
| 6 | 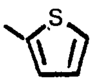 | 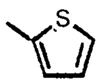 | $-CH_3$ | $-CH_3$ | a | 104-105 |
| 7 | 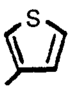 | 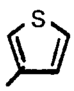 | $-CH_3$ | $-CH_3$ | a | 134-135 |
| 8 | 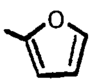 | 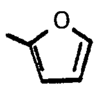 | $-CH_3$ | $-CH_3$ | a | 78-79 |
| 9 | 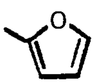 | 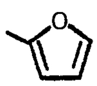 | $-CH_3$ | $-CH_3$ | e | 56-58 |
| 10 | 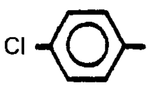 | 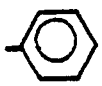 | $-CH_3$ | $-CH_3$ | e | 69-70 |
| 11 | 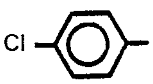 | 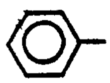 | H | $-CH_3$ | a | 111-112 |
| 12 | 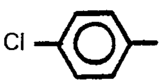 | 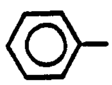 | H | $-CH_3$ | e | HCl-Salz 221-222 |
| 13 | 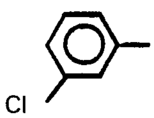 | 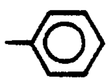 | $-CH_3$ | $-CH_3$ | a | Öl |
| 14 | 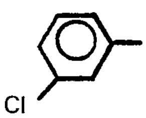 | 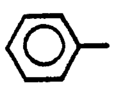 | $-CH_3$ | $-CH_3$ | e | Öl |
| 15 | 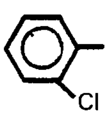 | 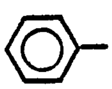 | $-CH_3$ | $-CH_3$ | a | 67-68 |
| 16 | 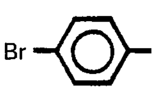 | 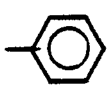 | $-CH_3$ | $-CH_3$ | a | 107 |
| 17 | 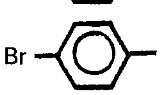 | 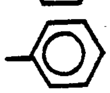 | $-CH_3$ | $-CH_3$ | e | 80-81 |

| Beispiel Nr. | $R_1$ | $R_2$ | $R_3$ | $R_4$ | Stellung d. Aminogruppe | Fp. °C |
|---|---|---|---|---|---|---|
| 18 | 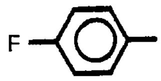 | 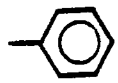 | $-CH_3$ | $-CH_3$ | a | 90-91 |
| 19 | 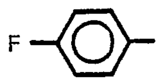 | 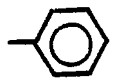 | $-CH_3$ | $-CH_3$ | e | Base Öl HCl-Salz 279-280 |
| 20 | 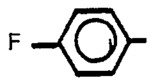 | 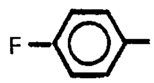 | $-CH_3$ | $-CH_3$ | a | 120-121 |
| 21 | 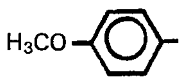 | 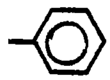 | $-CH_3$ | $-CH_3$ | a | 78-80 |
| 22 | 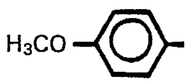 | 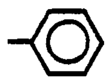 | $-CH_3$ | $-CH_3$ | e | 105-106 |
| 23 | 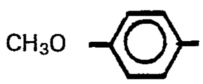 | 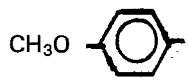 | $-CH_3$ | $-CH_3$ | a | 122-123 |
| 24 | 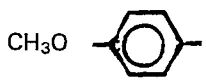 | 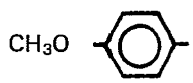 | $-CH_3$ | $-CH_3$ | e | 129 |
| 25 | 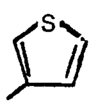 | 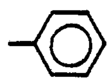 | $-CH_3$ | $-CH_3$ | a | 75-76 |
| 26 | 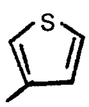 | 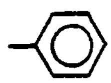 | $-CH_3$ | $-CH_3$ | e | HCl-Salz 265-266 |
| 27 | 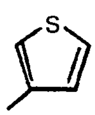 | 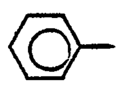 | H | $-CH_3$ | a | HCl-Salz 250 |
| 28 | 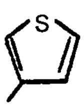 | 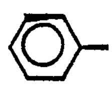 | H | $-CH_3$ | e | HCL-Salz 245 |
| 29 | 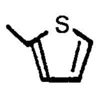 | 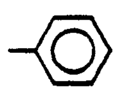 | $-CH_3$ | $-CH_3$ | a | 79-80 |
| 30 | 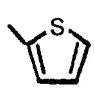 | 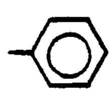 | $-CH_3$ | $-CH_3$ | e | Base 100-101 HCl-Salz 250-251 |
| 31 | 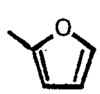 | 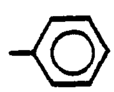 | $-CH_3$ | $-CH_3$ | a | 52-53 |
| 32 | 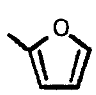 | 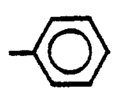 | $-CH_3$ | $-CH_3$ | e | HCl-Salz 215-220 |
| 33 | 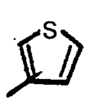 | 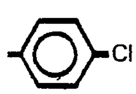 | $-CH_3$ | $-CH_3$ | a | Öl |

| Beispiel Nr. | $R_1$ | $R_2$ | $R_3$ | $R_4$ | Stellung d. Amino-gruppe | Fp. °C |
|---|---|---|---|---|---|---|
| 34 | 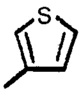 | 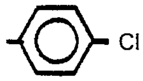 | $-CH_3$ | $-CH_3$ | e | Base Öl<br>HCl-Salz<br>235 |
| 35 | 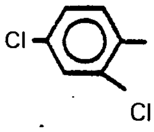 | 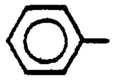 | $-CH_3$ | $-CH_3$ | a | Öl |
| 36 | 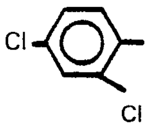 | 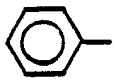 | $-CH_3$ | $-CH_3$ | e | Öl |
| 37 | 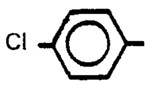 | 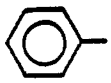 | $-C_2H_5$ | $-C_2H_5$ | a | 120-121 |
| 38 | 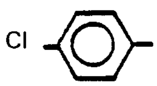 | 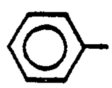 | $-C_2H_5$ | $-C_2H_5$ | e | Öl |
| 39 | 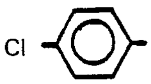 | 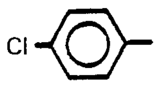 | H | $CH_3$<br>$-CH$<br>$CH_3$ | a | 128-129 |
| 40 | 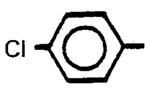 | 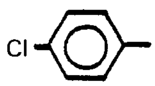 | H | $CH_3$<br>$-CH$<br>$CH_3$ | e | 109-110 |
| 41 | 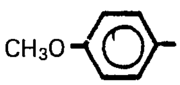 | 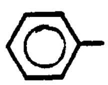 | H | $-CH_3$ | a | 115<br>HCl-Salz<br>232 |
| 42 | 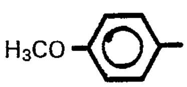 | 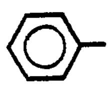 | H | $-CH_3$ | e | HCl-Salz<br>211 |
| 43 | 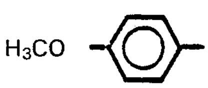 | 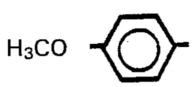 | H | $-CH_3$ | a | HCl-Salz<br>263 |
| 44 | 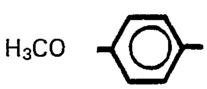 | 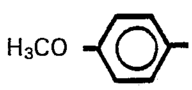 | H | $-CH_3$ | e | HCl-Salz<br>220 |
| 45 | 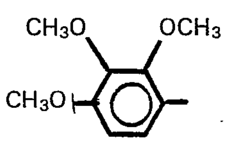 | 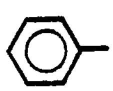 | $-CH_3$ | $-CH_3$ | a | 115<br>HCl-Salz<br>237 |
| 46 | 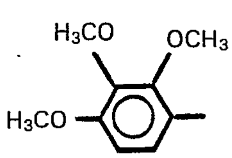 | 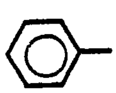 | $-CH_3$ | $-CH_3$ | e | 191-192<br>HCl-Salz<br>232 |

| Beispiel Nr. | $R_1$ | $R_2$ | $R_3$ | $R_4$ | Stellung d. Aminogruppe | Fp. °C |
|---|---|---|---|---|---|---|
| 47 | 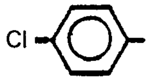 | 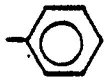 | 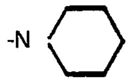 | | a | 164-165 |
| 48 | 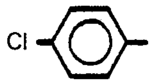 | 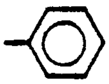 | 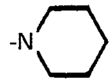 | | e | Öl |
| 49 | 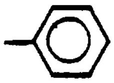 | 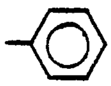 | 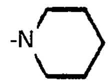 | | a | 183-184 |
| 50 | 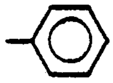 | 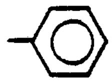 | 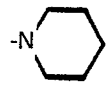 | | e | HCl-Salz > 260 |
| 51 | 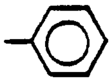 | 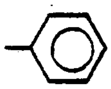 | 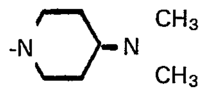 | | a | HCl-Salz 304-305 |
| 52 | 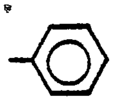 | 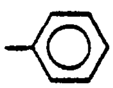 | 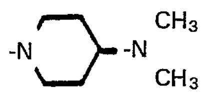 | | e | HCl-Salz > 310 |
| 53 | 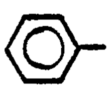 | 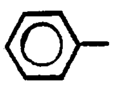 | 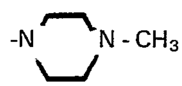 | | a | 134-135 |
| 54 | 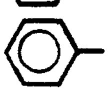 | 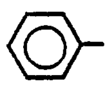 | 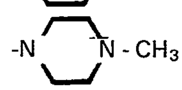 | | e | HCl-Salz 255-257 |

*Formulierungsbeispiele*

a) *Dragees*

1 Drageekern enthält:

| | |
|---|---|
| Wirkstoff gemäss der Erfindung | 25,0 mg |
| Milchzucker | 50,0 mg |
| Maisstärke | 22,0 mg |
| Gelatine | 2,0 mg |
| Magnesiumstearat | 1,0 mg |
| | 100,0 mg |

*Herstellung:*

Die Mischung der Wirksubstanz mit Milchzucker und Maisstärke wird mit einer 10%igen wässrigen Gelatinelösung durch ein Sieb mit 1 mm Maschenweite granuliert, bei 40°C getrocknet und nochmals durch ein Sieb getrieben. Das so erhaltene Granulat wird mit Magnesiumstearat gemischt und verpresst. Die so erhaltenen Kerne werden in üblicher Weise mit einer Hülle überzogen, die mit Hilfe einer wässrigen Suspension von Zucker, Titandioxyd, Talkum und Gummi arabicum aufgebracht wird. Die fertigen Dragees werden mit Bienenwachs poliert.

Dragee-Endgewicht: 200 mg

b) *Tabletten*

| | |
|---|---|
| Wirkstoff gemäss der Erfindung | 10,0 mg |
| Milchzucker | 40,0 mg |
| Maisstärke | 44,0 mg |
| lösliche Stärke | 5,0 mg |
| Magnesiumstearat | 1,0 mg |
| | 100,0 mg |

*Herstellung:*

Wirkstoff und Magnesiumstearat werden mit einer wässrigen Lösung der löslichen Stärke granuliert, das Granulat getrocknet und innig mit Milchzucker und Maisstärke vermischt. Das Gemisch wird sodann zu Tabletten von 100 mg Gewicht verpresst, die je 10 mg Wirkstoff enthalten.

c) *Suppositorien*

1 Zäpfchen enthält:

| | |
|---|---|
| Wirkstoff gemäss der Erfindung | 10,0 mg |
| Zäpfchenmasse | 1.690,0 mg |

*Herstellung:*

Die feingepulverte Substanz wird mit Hilfe eines Eintauch-Homogenisators in die geschmolzene und auf 40°C abgekühlte Zäpfchenmasse eingerührt. Die Masse wird bei 35°C in leicht vorgekühlte Formen gegossen.

d) *Ampullen*

| Zusammensetzung: | Gew.-Teile |
|---|---|
| Wirkstoff gemäss der Erfindung | 5,0 |
| Natriumpyrosulfit | 1,0 |
| Dinatriumsalz der Äthylendiamintetraessigsäure | 0,5 |

| | |
|---|---|
| Natriumchlorid | 8,5 |
| doppelt destilliertes Wasser | as 1000,0 |

*Herstellung:*

Der Wirkstoff und die Hilfsstoffe werden in einer ausreichenden Menge Wasser gelöst und mit der notwendigen Menge Wasser auf die gewünschte Konzentration gebracht. Die Lösung wird filtriert und unter aseptischen Bedingungen in 1 ml Ampullen abgefüllt. Zuletzt werden die Ampullen sterilisiert und verschlossen.

Jede Ampulle enthält 5,0 mg Wirkstoff.

**Patentansprüche für die Vertragsstaaten:**
BE, CH, DE, FR, IT, LI, LU, GB, SE

1. Substituierte 4-Amino-2,6-diaryl-tetrahydrothiopyrane der allgemeinen Formeln

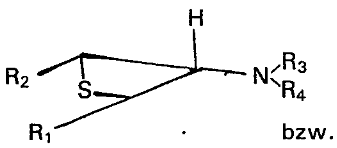

(I)

bzw.

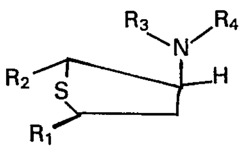

(Ia)

worin die Reste

$R_1$ und $R_2$, die gleich oder verschieden sein können, Phenyl, ein-, zwei- oder dreifach durch ein Halogenatom, eine Methyl- oder Methoxygruppe substituiertes Phenyl, Thienyl oder Furyl bedeuten,

$R_3$ für Wasserstoff oder eine geradkettige oder verzweigte Alkylgruppe mit 1-3 Kohlenstoffatomen und

$R_4$ für eine geradkettige oder verzweigte Alkylgruppe mit 1-3 Kohlenstoffatomen steht oder beide Reste $R_3$ und $R_4$ zusammen mit dem Stickstoffatom einen Piperidin-, Piperazin- oder Morpholinoring bilden, wobei der Piperidinring gegebenenfalls in 4-Stellung durch eine Amino- oder Dimethyl-Aminogruppe weiter substituiert ist oder der Piperazinring in 4-Stellung eine Alkylgruppe mit 1-2 Kohlenstoffatomen aufweisen kann, und deren physiologisch verträgliche Säureadditionssalze.

2. 4-Mono- und Dialkylamino-2,6-diaryl-tetrahydrothiopyrane der allgemeinen Formeln I bzw. Ia gemäss Anspruch 1, worin $R_1$ und $R_2$ gleich oder verschieden sein können und Phenyl, ein-, zwei- oder dreifach durch ein Halogenatom, eine Methyl- oder Methoxygruppe substituiertes Phenyl, Thienyl oder Furyl bedeuten und deren physiologisch verträgliche Säureadditionssalze.

3. 4a-Dimethylamino-2,6-bis-(4-methoxyphenyl)-tetrahydrothiopyran und seine physiologisch verträglichen Säureadditionssalze.

4. 4e-Dimethylamino-2,6-bis-(4-methoxyphenyl)-tetrahydrothiopyran und seine physiologisch verträglichen Säureadditionssalze.

5. 4a-Methylamino-2,6-bis-(4-methoxyphenyl)--tetrahydrothiophen und seine physiologisch verträglichen Säureadditionssalze.

6. 4e-Methylamino-2,6-bis-(4-methoxyphenyl)--tetrahydrothiopyran und seine physiologisch verträglichen Säureadditionssalze.

7. 4a-Dimethylamino-2-(4-chlorphenyl)-6-phenyl-tetrahydrothiopyran und seine physiologisch verträglichen Säureadditionssalze.

8. 4e-Dimethylamino-2-(4-chlorphenyl)-6-phenyl-tetrahydrothiopyran und seine physiologisch verträglichen Säureadditionssalze.

9. 4a-Dimethylamino-2-(4-methoxyphenyl)-6--phenyl-tetrahydrothiopyran und seine physiologisch verträglichen Säureadditionssalze.

10. 4e-Dimethylamino-2-(4-methoxyphenyl)-6--phenyl-tetrahydrothiopyran und seine physiologisch verträglichen Säureadditionssalze.

11. Verfahren zur Herstellung von 4-Amino-2,6--diaryl-tetrahydrothiopyranen der allgemeinen Formeln I und Ia gemäss Anspruch 1, dadurch gekennzeichnet, dass man ein Tetrahydrothiopyran der allgemeinen Formel

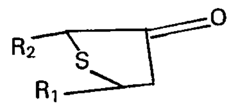

(II)

worin die Reste

$R_1$ und $R_2$ die in Anspruch 1 angegebene Bedeutung besitzen, mit einem geeigneten Reduktionsmittel zu den beiden entsprechenden stereoisomeren Carbinolen der allgemeinen Formeln

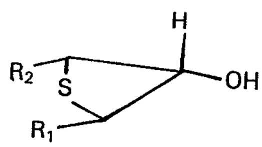

bzw.

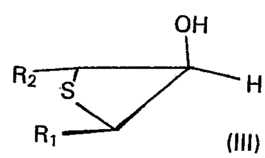

(III) (IIIa)

worin die Reste

$R_1$ und $R_2$ die in Anspruch 1 angegebene Bedeutung besitzen, umsetzt, diese Verbindungen mit Tosyl- oder Mesylhalogenid verestert, die Ester anschliessend mit einem primären oder einem sekundären Amin behandelt, die so erhaltenen Endprodukte der Formeln I und Ia einer üblichen Isomerentrennung unterwirft und die Isomeren gegebenenfalls in bekannter Weise in ihre Säureadditionssalze überführt.

12. Verfahren nach Anspruch 11, dadurch gekennzeichnet, dass man zur Herstellung von 4-Mono- bzw. 4-Dialkylamino-2,6-diaryl-tetrahydrothiopyranen ein Thiopyranon der allgemeinen Formel

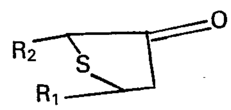

(II)

worin

$R_1$ und $R_2$ die oben angeführte Bedeutung besitzen, in Gegenwart eines komplexen Hydrides oder in Gegenwart von Wasserstoff und einem Katalysator oder aber bei Temperaturen von 0 bis —10°C in Gegenwart von Ameisensäure mit einem Amin der allgemeinen Formel

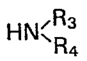

(IV)

worin

$R_3$ Wasserstoff oder eine geradkettige oder verzweigte Alkylgruppe mit 1-3 Kohlenstoffatomen und

$R_4$ eine geradkettige oder verzweigte Alkylgruppe mit 1-3 Kohlenstoffatomen bedeuten, umsetzt.

13. Verfahren nach Anspruch 12, dadurch gekennzeichnet, dass man eine Verbindung der allgemeinen Formel

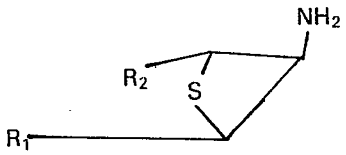

(V)

worin die Reste $R_1$ und $R_2$ die oben angeführte Bedeutung besitzen, in an sich bekannter Weise mono- oder dialkyliert.

14. Pharmazeutische Zubereitungen, enthaltend als Wirkstoff eine oder mehrere Verbindungen der allgemeinen Formeln I bzw. Ia gemäss Anspruch 1 oder deren physiologisch unbedenkliche Säureadditionssalze in Kombination mit üblichen Hilfs- und/oder Trägerstoffen.

15. Verfahren zur Herstellung von pharmazeutischen Präparaten gemäss Anspruch 14, dadurch gekennzeichnet, dass man Verbindungen der allgemeinen Formeln I bzw. Ia mit üblichen galenischen Hilfs- und/oder Trägerstoffen zu üblichen pharmazeutischen Anwendungsformen verarbeitet.

**Patentansprüche für den Vertragsstaat:** AT

1. Verfahren zur Herstellung neuer 4-Amino-2,6-diaryl-tetrahydrothiopyrane der allgemeinen Formeln

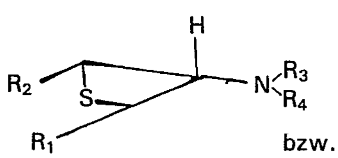

(I)

bzw.

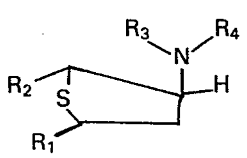

(Ia)

worin die Reste

$R_1$ und $R_2$, die gleich oder verschieden sein können, Phenyl, ein-, zwei- oder dreifach durch ein Halogenatom, eine Methyl- oder Methoxygruppe substituiertes Phenyl, Thienyl oder Furyl bedeuten,

$R_3$ für Wasserstoff oder eine geradkettige oder verzweigte Alkylgruppe mit 1-3 Kohlenstoffatomen und

$R_4$ für eine geradkettige oder verzweigte Alkylgruppe mit 1-3 Kohlenstoffatomen steht oder beide Reste $R_3$ und $R_4$ zusammen mit dem Stickstoffatom einen Piperidin-, Piperazin- oder Morpholinoring bilden, wobei der Piperidinring gegebenenfalls in 4-Stellung durch eine Amino- oder Dimethyl-Aminogruppe weiter substituiert ist oder der Piperazinring in 4-Stellung eine Alkylgruppe mit 1-2 Kohlenstoffatomen aufweisen kann, dadurch gekennzeichnet, dass man ein Tetrahydrothiopyran der allgemeinen Formel

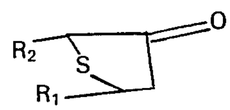

(II)

worin die Reste

$R_1$ und $R_2$ die in Anspruch 1 angegebene Bedeutung besitzen, mit einem geeigneten Reduktionsmittel zu den beiden entsprechenden stereoisomeren Carbinolen der allgemeinen Formeln

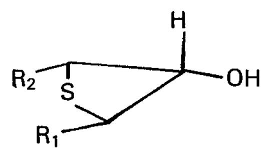

(III)

bzw.

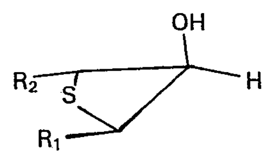

(IIIa)

worin die Reste

$R_1$ und $R_2$ die oben angegebene Bedeutung besitzen, umsetzt, diese Verbindungen mit Tosyl- oder Mesylhalogenid verestert, die Ester anschliessend mit einem primären oder einem sekundären Amin behandelt, die so erhaltenen Endprodukte der Formeln I und Ia einer üblichen Isomerentrennung unterwirft und die Isomeren gegebenenfalls in bekannter Weise in ihre Säureadditionssalze überführt.

2. Verfahren nach Anspruch 1, dadurch gekennzeichnet, dass man zur Herstellung von 4-Mono- bzw. 4-Dialkylamino-2,6-diaryl-tetrahydrothiopyranen ein Thiopyranon der allgemeinen Formel

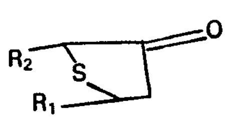

(II)

worin

$R_1$ und $R_2$ die oben angeführte Bedeutung besitzen, in Gegenwart eines komplexen Hydrides oder in Gegenwart von Wasserstoff und einem Katalysator oder aber bei Temperaturen von 0 bis —10°C in Gegenwart von Ameisensäure mit einem Amin der allgemeinen Formel

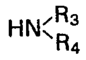

(IV)

worin

$R_3$ Wasserstoff oder eine geradkettige oder verzweigte Alkylgruppe mit 1-3 Kohlenstoffatomen und

$R_4$ eine geradkettige oder verzweigte Alkylgruppe mit 1-3 Kohlenstoffatomen bedeuten, umsetzt.

3. Verfahren nach Anspruch 2, dadurch gekennzeichnet, dass man eine Verbindung der allgemeinen Formel

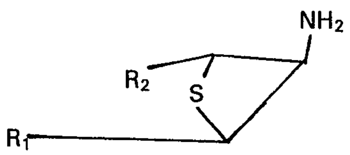

(V)

worin die Reste $R_1$ und $R_2$ die oben angeführte Bedeutung besitzen, in an sich bekannter Weise mono- oder dialkyliert.

**Claims for the Contracting States:**
BE, CH, DE, FR, IT, LI, LU, GB, SE

1. Substituted 4-amino-2,6-diaryl-tetrahydrothiopyrans of general formula

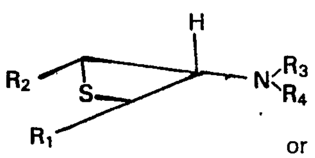

(I)

or

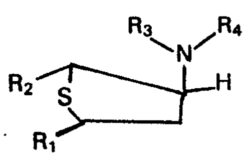

(Ia)

the groups

$R_1$ and $R_2$, which may be the same or different, represent phenyl or a phenyl, which is mono-, di- or trisubstituted by a halogen atom or by a methyl or methoxy group, thienyl or furyl,

$R_3$ represents hydrogen or a straight-chained or branched alkyl group with 1 to 3 carbon atoms and

$R_4$ represents a straight-chained or branched alkyl group with 1 to 3 carbon atoms or the two groups $R_3$ and $R_4$ together with the nitrogen atom form a piperidine, piperazine or morpholino ring, the piperidine ring optionally being further substituted in the 4 position by an amino or dimethylamino group, whilst the piperazine ring may comprise, in the 4 position, an alkyl group with 1 to 2 carbon atoms, and the physiologically acceptable acid addition salts thereof.

2. 4-Mono- and dialkylamino-2,6-diaryl-tetrahydrothiopyrans of general formula I or Ia as claimed in claim 1, wherein $R_1$ and $R_2$ may be the same or different and represent phenyl or a phenyl, thienyl or furyl group mono-, di- or trisubstituted by a halogen atom or by a methyl or methoxy group, and the physiologically acceptable acid addition salts thereof.

3. 4a-Dimethylamino-2,6-bis-(4-methoxyphenyl)-tetrahydrothiopyran and the physiologically acceptable acid addition salts thereof.

4. 4e-Dimethylamino-2,6-bis-(4-methoxyphenyl)-tetrahydrothiopyran and the physiologically acceptable acid addition salts thereof.

5. 4a-Methylamino-2,6-bis-(4-methoxyphenyl)-tetrahydrothiopyran and the physiologically acceptable acid addition salts thereof.

6. 4e-Methylamino-2,6-bis-(4-methoxyphenyl)-tetrahydrothiopyran and the physiologically acceptable acid addition salts thereof.

7. 4a-Dimethylamino-2-(4-chlorophenyl)-6-phenyl-tetrahydrothiopyran and the physiologically acceptable acid addition salts thereof.

8. 4e-Dimethylamino-2-(4-chlorophenyl)-6-phenyl-tetrahydrothiopyran and the physiologically acceptable acid addition salts thereof.

9. 4a-Dimethylamino-2-(4-methoxyphenyl)-6-phenyl-tetrahydrothiopyran and the physiologically acceptable acid addition salts thereof.

10. 4e-Dimethylamino-2-(4-methoxyphenyl)-6-phenyl-tetrahydrothiopyran and the physiologically acceptable acid addition salts thereof.

11. Process for preparing 4-amino-2,6-diaryl-tetrahydrothiopyrans of general formulae I and Ia as claimed in claim 1, characterised in that a tetrahydrothiopyran of general formula

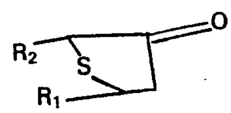

(II)

wherein the groups

$R_1$ and $R_2$ have the meanings given in claim 1, is reacted with a suitable reducing agent to form the two corresponding stereoisomeric carbinols of general formula

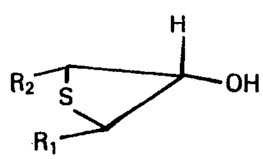

(III)

or

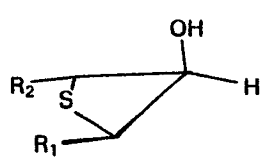

(IIIa)

wherein the groups

$R_1$ and $R_2$ have the meanings given in claim 1, these compounds are esterified with tosyl or mesyl halide, these esters are subsequently treated with a primary or secondary amine, the end products of formulae I and Ia thus obtained are subjected to conventional resolution of the isomers and the isomers are optionally converted into the acid addition salts thereof in known manner.

12. Process as claimed in claim 11, characterised in that, in order to prepare 4-mono- or 4-dialkylamino-2,6-diaryl-tetrahydrothiopyrans, a thiopyranone of general formula

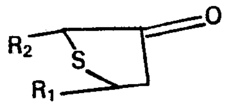

(II)

wherein

$R_1$ and $R_2$ are as hereinbefore defined, is reacted in the presence of a complex hydride or in the presence of hydrogen and a catalyst or at temperatures of from 0 to —10°C in the presence of formic acid with an amine of general formula

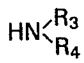

(IV)

wherein

$R_3$ represents hydrogen or a straight-chained or branched alkyl group with 1 to 3 carbon atoms and

$R_4$ represents a straight-chained or branched alkyl group with 1 to 3 carbon atoms.

13. Process as claimed in claim 12, characterised in that a compound of general formula

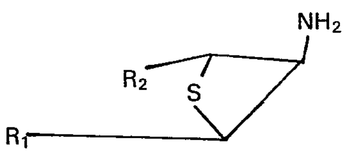

(V)

wherein the groups $R_1$ and $R_2$ are as hereinbefore defined, is mono- or dialkylated in a manner known per se.

14. Pharmaceutical preparations containing, as active substance, one or more compounds of general formula I or Ia as claimed in claim 1 or the physiologically acceptable acid addition salts thereof combined with conventional excipients and/or carriers.

15. Process for preparing pharmaceutical preparations as claimed in claim 14, characterised in that compounds of general formula I or Ia are processed with conventional galenic excipients and/or carriers to form conventional pharmaceutical preparations.

**Claims for the Contracting State:** AT

1. Process for preparing novel 4-amino-2,6-diaryl-tetrahydrothiopyrans of general formuale

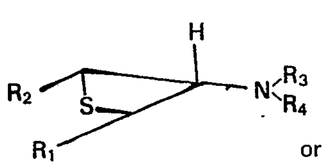

(I)

or

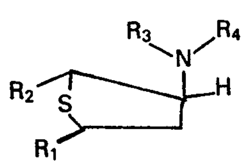

(Ia)

wherein the groups

$R_1$ and $R_2$, which may be the same or different, represent phenyl or a phenyl, which is mono-, di- or trisubstituted by a halogen atom or by a methyl or methoxy group, thienyl or furyl,

$R_3$ represents hydrogen or a straight-chained or branched alkyl group with 1 to 3 carbon atoms and

$R_4$ represents a straight-chained or branched alkyl group with 1 to 3 carbon atoms or the two groups $R_3$ and $R_4$ together with the nitrogen atom form a piperidine, piperazine or morpholino ring, the piperidine ring optionally being further substituted in the 4 position by an amino or dimethylamino group, whilst the piperazine ring may comprise, in the 4 position, an alkyl group with 1 to 2 carbon atoms, characterised in that a tetrahydrothiopyran of general formula

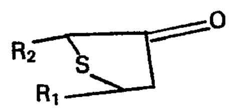

(II)

wherein the groups

$R_1$ and $R_2$ have the meanings given above, is reacted with a suitable reducing agent to form the two corresponding stereoisomeric carbinols of general formula

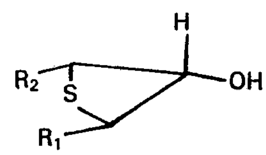

(III)

or

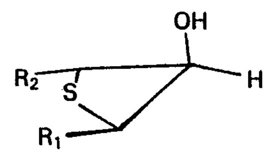

(IIIa)

wherein the groups

$R_1$ and $R_2$ have the meanings given above, these compounds are esterified with tosyl or mesyl halide, the esters are subsequently treated with a primary or secondary amine, the end products of formulae I and Ia thus obtained are subjected to conventional resolution of the isomers and the isomers are optionally converted into the acid addition salts thereof in known manner.

2. Process as claimed in claim 1, characterised in that, in order to prepare 4-mono- or 4-dialkylamino-2,6-diaryl-tetrahydrothiopyrans, a thiopyranone of general formula

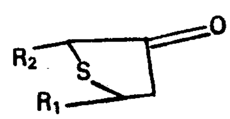

(II)

wherein

$R_1$ and $R_2$ are as hereinbefore defined, is reacted in the presence of a complex hydride or in the presence of hydrogen and a catalyst or at temperatures of from 0 to —10°C in the presence of formic acid with an amine of general formula

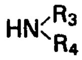

(IV)

wherein

$R_3$ represents hydrogen or a straight-chained or branched alkyl group with 1 to 3 carbon atoms and

$R_4$ represents a straight-chained or branched alkyl group with 1 to 3 carbon atoms.

3. Process as claimed in claim 2, characterised in that a compound of general formula

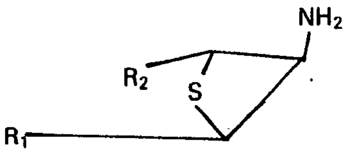

(V)

wherein the groups $R_1$ and $R_2$ are as hereinbefore defined, is mono- or dialkylated in a manner known per se.

**Revendications pour les Etats Contractants:**
BE, CH, DE, FR, IT, LI, LU, GB, SE

1. 4-amino-2,6-diaryl-tétrahydrothiopyrannes substitués de formules générales

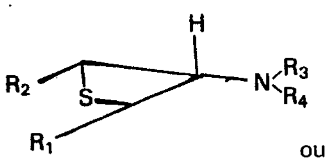

(I)

ou

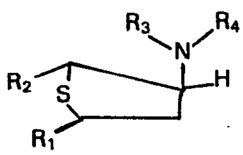

(Ia)

dans lesquelles les radicaux

$R_1$ et $R_2$, qui peuvent être identiques ou différents, représentent phényle, phényle substitué une, deux ou trois fois par un atome d'halogène, un groupe méthyle ou méthoxy, thiényle ou furyle,

$R_3$ représente l'hydrogène ou un groupe alcoyle rectiligne ou ramifié avec 1-3 atomes de carbone et

$R_4$ représente un groupe alcoyle rectiligne ou ramifié avec 1-3 atomes de carbone, ou les deux radicaux $R_3$ et $R_4$ forment ensemble avec l'atome d'azote un cycle pipéridine, pipérazine ou morpholino, le cycle pipéridine étant éventuellement encore substitué en position 4 par un groupe amino ou diméthylamino ou le cycle pipérazine pouvant présenter en position 4 un groupe alcoyle avec 1-2 atomes de carbone, et leurs sels d'addition d'acides physiologiquement supportables.

2. 4-mono- et dialcoylamino-2,6-diaryl-tétrahydrothiopyrannes de formules générales I ou respectivement Ia selon la revendication 1, dans lesquelles $R_1$ et $R_2$ peuvent être identiques ou différents et représentent phényle, phényle substitué une, deux ou trois fois par un atome d'halogène, un groupe méthyle ou méthoxy, thiényle ou furyle, et leurs sels d'addition d'acides physiologiquement supportables.

3. Le 4a-diméthylamino-2,6-bis-(4-méthoxyphényl)-tétrahydrothiopyranne et ses sels d'addition d'acides physiologiquement supportables.

4. Le 4e-diméthylamino-2,6-bis-(4-méthoxyphényl)-tétrahydrothiopyranne et ses sels d'addition d'acides physiologiquement supportables.

5. Le 4a-méthylamino-2,6-bis-(4-méthoxyphényl)-tétrahydrothiopyranne et ses sels d'addition d'acides physiologiquement supportables.

6. Le 4e-méthylamino-2,6-bis-(4-méthoxyphényl)-tétrahydrothiopyranne et ses sels d'addition d'acides physiologiquement supportables.

7. Le 4a-diméthylamino-2-(4-chlorophényl)-6-phényl-tétrahydrothiopyranne et ses sels d'addition d'acides physiologiquement supportables.

8. Le 4e-diméthylamino-2-(4-chlorophényl)-6-phényl-tétrahydrothiopyranne et ses sels d'addition d'acides physiologiquement supportables.

9. Le 4a-diméthylamino-2-(4-méthoxyphényl)-6-phényl-tétrahydrothiopyranne et ses sels d'addition d'acides physiologiquement supportables.

10. Le 4e-diméthylamino-2-(4-méthoxyphényl)-6-phényl-tétrahydrothiopyranne et ses sels d'addition d'acides physiologiquement supportables.

11. Procédé pour la préparation de 4-amino-2,6-diaryl-tétrahydrothiopyrannes de formules générales I et Ia selon la revendication 1, caractérisé en ce que l'on fait réagir un tétrahydrothiopyranne de formule générale

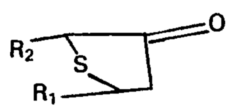

(II)

dans laquelle les radicaux

$R_1$ et $R_2$ possèdent la signification indiquée dans la revendication 1, avec un agent de réduction approprié pour donner les deux carbinols stéréoisomères correspondants de formules générales

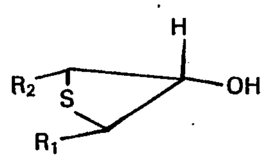

(III)

ou

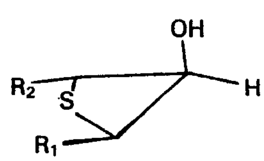

(IIIa)

dans lesquelles les radicaux

$R_1$ et $R_2$ possèdent la signification indiquée dans la revendication 1, en ce qu'on estérifie ces composés avec un halogénure de tosyle ou de mésyle, on traite

ensuite l'ester avec une amine primaire ou une amine secondaire, on soumet les produits finals ainsi obtenus de formules I et Ia à une séparation d'isomères habituelle et on transforme éventuellement les isomères de manière connue en leurs sels d'addition d'acides.

12. Procédé selon la revendication 11, caractérisé en ce que pour la préparation de 4-mono- ou respectivement 4-dialcoylamino-2,6-diaryl-tétrahydrothiopyrannes, on fait réagir une thiopyranone de formule générale

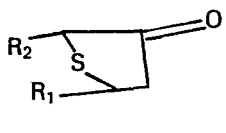

(II)

dans laquelle

$R_1$ et $R_2$ possèdent la signification indiquée plus haut, en présence d'un hydrure complexe ou en présence d'hydrogène et d'un catalyseur ou cependant à des températures de 0 à —10°C en présence d'acide formique avec une amine de formule générale

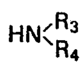

(IV)

$R_3$ représente l'hydrogène ou un groupe alcoyle rectiligne ou ramifié avec 1-3 atomes de carbone et

$R_4$ représente un groupe alcoyle rectiligne ou ramifié avec 1-3 atomes de carbone.

13. Procédé selon la revendication 12, caractérisé en ce qu'on mono- ou dialcoyle de manière connue en soi un composé de formule générale

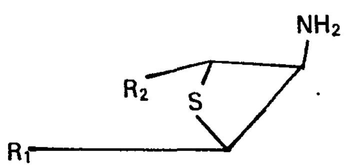

(V)

dans laquelle les radicaux $R_1$ et $R_2$ possèdent la signification indiquée plus haut.

14. Préparations pharmaceutiques contenant en tant que substance active un ou plusieurs composés de formules générales I ou respectivement Ia selon la revendication 1, ou leurs sels d'addition d'acides physiologiquement inoffensifs, en combinaison avec des adjuvants et/ou excipients habituels.

15. Procédé pour la fabrication de préparations pharmaceutiques selon la revendication 14, caractérisé en ce qu'on transforme des composés de formules générales I ou respectivement Ia, au moyen d'adjuvants et/ou excipients galéniques usuels, en des formes d'utilisation pharmaceutiques habituelles.

**Revendications pour l'Etat Contractant:** AT

1. Procédé pour la préparation de 4-amino-2,6-diaryl-tétrahydrothiopyrannes nouveaux de formules générales

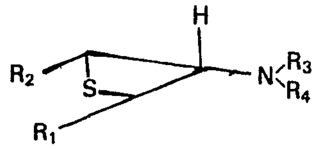

(I)

ou

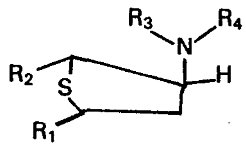

(Ia)

dans lesquelles les radicaux

$R_1$ et $R_2$, qui peuvent être identiques ou différents, représentent phényle, phényle substitué une, deux ou trois fois par un atome d'halogène, un groupe méthyle ou méthoxy, thiényle ou furyle,

$R_3$ représente l'hydrogène ou un groupe alcoyle rectiligne ou ramifié avec 1-3 atomes de carbone et

$R_4$ représente un groupe alcoyle rectiligne ou ramifié avec 1-3 atomes de carbone, ou les deux radicaux $R_3$ et $R_4$ forment ensemble avec l'atome d'azote un cycle pipéridine, pipérazine ou morpholino, le cycle pipéridine étant éventuellement encore substitué en position 4 par un groupe amino ou diméthylamino ou le cycle pipérazine pouvant présenter en position 4 un groupe alcoyle avec 1-2 atomes de carbone, caractérisé en ce que l'on fait réagir un tétrahydrothiopyranne de formule générale

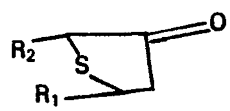

(II)

dans laquelle les radicaux

$R_1$ et $R_2$ possèdent la signification indiquée dans la revendication 1, avec un agent de réduction approprié pour donner les deux carbinols stéréoisomères correspondants de formules générales

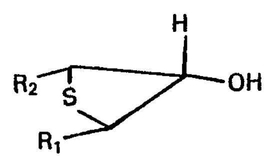

(III)

ou

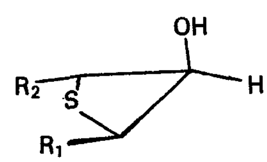

(IIIa)

dans lesquelles les radicaux

$R_1$ et $R_2$ possèdent la signification indiquée dans la revendication 1, en ce qu'on estérifie ces composés avec un halogénure de tosyle ou de mésyle, on traite ensuite l'ester avec une amine primaire ou une amine secondaire, on soumet les produits finals ainsi obtenus de formules I et Ia à une séparation d'isomères habituelle et on transforme éventuellement les isomères de manière connue en leurs sels d'addition d'acides.

2. Procédé selon la revendication 1, caractérisé en ce que pour la préparation de 4-mono- ou respectivement 4-dialcoylamino-2,6-diaryl-tétrahydrothiopyrannes, on fait réagir une thiopyranone de formule générale

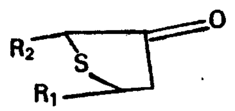

(II)

dans laquelle

$R_1$ et $R_2$ possèdent la signification indiquée plus haut, en présence d'un hydrure complexe ou en présence d'hydrogène et d'un catalyseur ou cependant à des températures de 0 à —10°C en présence d'acide formique avec une amine de formule générale

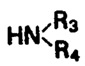

(IV)

dans laquelle

$R_3$ représente l'hydrogène ou un groupe alcoyle rectiligne ou ramifié avec 1-3 atomes de carbone et

$R_4$ représente un groupe alcoyle rectiligne ou ramifié avec 1-3 atomes de carbone.

3. Procédé selon la revendication 2, caractérisé en ce qu'on mono- ou dialcoyle de manière connue en soi un composé de formule générale

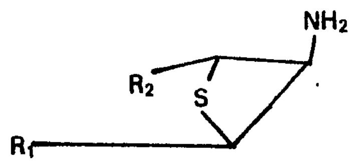

(V)

dans laquelle les radicaux $R_1$ et $R_2$ possèdent la signification indiquée plus haut.